# EUROPEAN PATENT SPECIFICATION

(11) **EP 1 940 340 B1**
(45) Date of publication and mention of the grant of the patent: **21.05.2014**
(21) Application number: 06806367.6
(22) Date of filing: 18.10.2006
(51) Int. Cl.: A61K 6/00, A61K 6/083

(54) **DENTAL ADHESIVE**
DENTALKLEBER
ADHÉSIF DENTAIRE

(30) Priority: 20.10.2005 EP 05022930
(43) Date of publication of application: 09.07.2008
(73) Proprietor: Dentsply DeTrey GmbH, 78467 Konstanz (DE)
(72) Inventor: KLEE, Joachim, 78315 Radolfzell (DE); LEHMANN, Uwe, 78467 Konstanz (DE)
(74) Representative: Hartz, Nikolai
(86) International application number: PCT/EP2006/010051
(87) International publication number: WO 2007/045459

(56) References cited:
- EP-A- 1 548 021
- WO-A-02/48213
- US-A- 5 320 886

## Description

### Field of the invention

The present invention relates to a dental adhesive having improved storage stability and low toxicity. The dental adhesive may be a one-pack dental adhesive composition, in particular a total etch dental adhesive. The present invention also relates to the use of a specific thermal polymerization inhibitor in a dental adhesive composition.

### Background of the invention

Dental adhesive compositions known from the prior art typically contain a mixture of a polymerizable monomer and an initiator system in a suitable solvent. The activity of the polymerizable monomers and the initiator system of the mixture must be adapted to provide sufficient curing activity and adhesion on dentin and enamel surfaces. However, an increased activity leads to a complex stability problem during storage of the components of the mixture. Specifically, the initiator system may be activated leading to premature polymerization of the mixture.

As a result of the stability problem of the mixture, the storage stability at room temperature of commercial dental adhesive compositions known from the prior art may be insufficient. For example, conventional commercial one-part self-etching, self-priming dental adhesive compositions must be stored in a refrigerator in order to avoid deterioration by solvolysis or polymerization. The commercial composition "iBond Gluma inside" may be mentioned, which has a low thermal stability when stored at temperatures of 37°C or 50°C due to premature polymerization within less than two weeks, which is indicative of an insufficient thermal stability at room temperature for all practical purposes. Similar stability problems are observed with other dental adhesive compositions.

EP-A 1 548 021 suggests hydrolysis stable one-part self-etching, self-priming dental adhesive compositions containing specific monomers having improved resistance against hydrolysis under acidic conditions. In order to improve the stability of the initiator system, EP-A 1 548 021 suggests a stabilizer such as hydroquinone monomethylether, 2,6-di-tert.-butyl-p-cresol, tetramethyl piperidine N-oxyl radical and galvanoxyl radical. However, generic one-part self-etching, self-priming dental adhesive composition known from EP-A 1 548 021 still require improvement of the thermal stability at storage for attaining a stability of at least 10 days at 60 °C required. Moreover, hydroquinone is an allergenic compound imparting undesirable toxic properties to a dental adhesive composition.

### Summary of the Invention

It is a problem of the present invention to provide a dental adhesive composition having a low toxicity and thermal stability at storage of at least 10 days at 60 °C.

Moreover, it is the problem of the present invention to provide a specific class of compounds which may be used to stabilize a dental adhesive composition for at least 20 days during storage at 60 °C.

The present invention provides a non-aqueous dental adhesive comprising a mixture containing
(i) one or more polymerizable monomers optionally containing an acidic group,
(ii) a polymerization initiator,
(iii) a thermal polymerization inhibitor of the following formula (I): 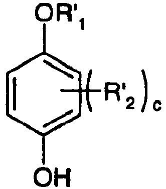 wherein
   R'₁ represents
      a hydrogen atom, or a saturated hydrocarbon group having 1 to 18 carbon atoms.
   R'₂, is a tert.-butyl group,
   and c represents an integer of from 1 to 4, and
**(iv) optionally an organic solvent.**

The present invention is based on the recognition that a mixture containing one or more polymerizable monomers optionally containing an acidic group, one or more organic or inorganic acids, and a polymerization initiator is problematic with regard to polymerization whereby conventional stabilizers such as hydroquinone monomethylether, 2,6-di-tert.-butyl-p-cresol, tetramethyl piperidine N-oxyl radical and galvanoxyl radical provide an insufficient effect for attaining a high storage stability.

The present invention is furthermore based on the recognition that a specific class of water insoluble stabilizers provides a surprising stabilizing effect so that a dental adhesive may be provided which has an excellent storage stability due to an improved resistance against premature polymerization.

Accordingly, the present invention also relates to the use of a compound of the following formula (I): 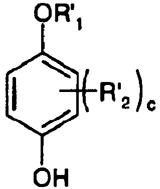 wherein
R'₁ represents
   a hydrogen atom, or a saturated hydrocarbon group having 1 to 18 carbon atoms.
R'₂, is a tert.-butyl group
   and c represents an integer of from 1 to 4,
as a thermal polymerization inhibitor In a dental composition.

### Description of the preferred embodiments

The dental adhesive composition according to the present invention contains a water-insoluble thermal polymerization inhibitor of formula (I). Preferably, the saturated hydrocarbon group which may be present as R'₁ or R'₂ in formula (I) represents a straight chain or branched C₁₋₁₈ alkyl group or a C₃₋₁₈ cycloalkyl group optionally substituted by one or more C₁₋₅ alkyl groups or a C₄₋₁₈ cycloalkylalkyl group optionally substituted by one or more C₁₋₅ alkyl groups.

Preferably, R'₁ represents a straight chain or branched C₁₋₁₈ alkyl group. In a preferred embodiment, R'₁ is hydrogen or a tert.-butyl group.

R'₂ in formula (I) is believed to provide a steric effect due to the bulky nature of the substituent in this position. R'₂ is a tert.-butyl group. c represents an integer of from 1 to 4, preferably 1 or 2. In a specific embodiment, c is 1.

Preferably, thermal polymerization inhibitor is a compound of the following formula (I'): 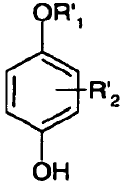 wherein
R'₁ represents a hydrogen atom, or a saturated hydrocarbon group having 1 to 18 carbon atoms; R'₂ is a tert.-butyl group. Most preferably, the inhibitor is tert.-butyl hydroquinone (TBHQ) or tert.-butyl hydroxyanisole (BHA). Preferably, the initiator is contained in the dental adhesive composition in an amount of from 0.01 to 0.5 mol%, more preferably in an amount of from 0.05 to 0.3 mol%.

The dental adhesive composition according to the present invention contains polymerizable monomers optionally containing an acidic group.

The polymerizable monomers in the dental adhesive composition according to the invention are capable of free-radical polymerization and are preferably (meth)acrylate monomers or oligomers. The dental adhesive according to the invention may contain a polymerizable monomer or oligomer as a mixture of different compounds or as isomers of the same compound. The polymerizable monomer or oligomer may include a derivative of at least one unsaturated carboxylic acid selected from the group consisting of acrylic acid, methacrylic acid, cyanoacrylic acid and itaconic acid, and mixtures thereof, a derivative of styrene, or a polymerizable moiety containing a carbon-carbon double bond conjugated with a carbonyl group.

The (meth)acrylate monomer or oligomer is selected from materials having at least one, and preferably two to four polymerizable double bonds per molecule so that the cured dental adhesive be crosslinked and thus better suited for use in the oral cavity. Monomers with a single polymerizable double-bond may be used in order to adjust the viscosity of the composition. (Meth)acrylate monomer materials useful herein are well known in the art. The preferred materials generally include monomers having a central portion containing an organic moiety and at least two (meth)acrylic end groups. Desirable characteristics for such monomers and/or oligomers include good film forming properties, low viscosity, low polymerization shrinkage, low water sorption and the ability to cure rapidly and completely in the mouth. It is also desirable that the monomers be low in volatility and non-irritating to the tooth pulp. A mixture of two or more appropriate methacrylate monomers is within the scope of this invention. In fact, depending on the choice of monomers, mixture are often highly desirable to optimize the characteristics of the resulting dental composition.

The polymerizable monomer or oligomer may also be selected from the group consisting of hydroxyethyl acrylate, hydroxypropyl acrylate, diurethane dimethacrylate resin, hydroxyethyl methacrylate, hydroxypropyl methacrylate, trimethylolpropane triacrylate, 1,6-hexanediacrylate, glycerin diacrylate, triethyleneglycol diacrylate, tetraethyleneglycol diacrylate, and 2-acrylamido-2-methyl-1-propansulfonic acid, a reaction product of butane tetracarboxylic acid dianhydride and hydroxyethylmethacrylate, triethyleneglycol dimethacrylate, urethane dimethacrylate, and a reaction product of butane tetracarboxylic acid dianhydride and glycerol dimethacrylate, or acrylamides or derivatives thereof such as 2-acrylamido-2-methylpropane sulphonic acid, N,N-methylene-bis-acrylamide, N,N-ethylene-bis-acrylamide, and 1,3-bis(acrylamido)-N,N-diethylpropane.

The polymerizable monomer or oligomer may be a phosphate based acid adhesion promoter selected from the group consisting of phosphate ester or phosphonate derivatives of radical polymerizable alcohol or polyol derivatives. The phosphate ester derivatives may be prepared using the method given in US 4,514,342. As examples of suitable carboxylic acid based adhesion promoters may be mentioned the reaction product between butanetetracarboxylic acid dianhydride and hydroxylethyl acrylate as in US 5,218,070. Various radical polymerizable acidic monomers useful as adhesion promoters may also be obtained by many other means, for instance as given in US 4,806,381 and US 6,350,839.

The polymerizable monomer or oligomer may further be a carboxylic acid based adhesion promoter selected from the group consisting of reaction products between acid anhydrides and radical polymerizable derivatives of alcohols. The acid anhydride may be selected from the group consisting of butanetetracarboxylic acid dianhydride, tetrahydrofurantetracarboxylic acid dianhydride, benzenetetracarboxylic acid dianhydride and benzentricarboxylic acid anhydride. The radical polymerizable derivatives of alcohols may be selected from the group consisting of hydroxyethyl acrylate, hydroxypropyl-acrylate, glycerol diacrylate, pentaerythritol triacrylate, dipentaerythritol pentaacrylate, hexanediol acrylate, polyethylenoxide acrylate, and triallypentaerythritol.

These may be mono- or polyfunctional acrylates and methacrylates, of the kind described, for example, in EP-A-0 480 472. Moreover, functionalized monomers with terminal acrylate or methacrylate groups may likewise be used, of the kind described, e.g., in DE-A-2 312 559 and in EP-A-0 219 058.

The dental adhesive according to the present invention may contain polymerizable monomers in an amount of from 5 to 90 wt-%, preferably in an amount of from 20 to 70 wt.%.

It is preferred that the monomer or oligomer or monomer or oligomer blend has a viscosity of at most 100 Pas at 23°C, more preferably at most 5 Pas.

The dental adhesive composition according to the invention may optionally comprise an organic solvent. In a preferred embodiment, the dental adhesive contains one or more further solvents selected from conventional inert solvents such as short-chain alcohols, short-chain ketones, aliphatic or unsaturated ethers, and cyclic ethers conventionally used in the dental field. Preferred solvents are selected from acetone, ethanol and t-butanol. The dental adhesive composition may contain the solvent in an amount of from 10 to 95 wt.%.

The dental adhesive composition according to the present invention may further comprise an initiator. A chemical initiator may be used in case of a multicomponent composition. The chemical initiator is able to form radicals by mixing at least two chemically different substances, which have to be stored separately, without a further input of energy, which radicals are then able to initiate a polymerization reaction. Examples of such chemical initiator systems are peroxy amine or peroxy proton donor/metal compound mixtures, of the kind described by J. M. Antonucci et al. in J. Dental Research (1979), 58 (9), page 1887-1889 or in U.S. Pat. No. 5,166,117 and in EP-A-0 115 410, 0 115 948, 0 120 559 and 0 277 413. More preferred are initiators capable of forming radicals without mixing of different components, but based on input of energy such as thermal or light energy. In this case, the dental adhesive composition may be a one-component dental adhesive composition, wherein the initiator is a photoinitiator and/or a thermal initiator. The composition may comprise an alpha-diketone such as camphor quinone.

The dental adhesive may further contains an inorganic filler and/or an organic filler; preferably the filler is a nanofiller. A filler may be contained in an amount of from 0.5 to 20 wt.%, more preferably 2 to 10 wt.%.

A one-pack composition means that the composition of the present invention is contained in only one container which may be stored and allows application of the composition without any mixing and without any special equipment before the application. ,

A total etch composition is a one-pack composition having priming and bonding activity on a dental surface such as dentin or enamel.

The invention will now be further illustrated with reference to the following examples

### Examples

Test Formulation containing different inhibitors.

A series of test formulations containing different thermal polymerization inhibitors was prepared in order to illustrate the surprising thermal stability of a dental adhesive composition according to the present invention. The standard composition was used as follows:

| **Component** | **Content (wt.-%)** |
|---|---|
| BAP | 63.2 |
| BAA-TCD | 21.1 |
| DHPOBA analog | 54.2 |
| 2-Acrylamido-2-methyl-propanesulfonic acid (AMPS) | 43.8 |
| Camphor Quinone | 1.3 |
| TPO | 3.2 |
| DMABE | 1.5 |
| Total | 100.00 |
| | |
| Active Matrix | 55 |
| Acrylic acid | 9 |
| Water | 36 |
| Total | 100 |

The following comparative inhibitors were tested:
(i) hydroquinone (HQ),
(ii) hydroquinone monomethylether (HQME),
(iii) bisphenol A,
(iv) propyl gallate (PG)

The following inhibitors according to the present invention were tested:
(vii) tert-Butylhydroquinone (TBHQ), and
(viii) tert.-Butylhydroxyanisol (BHA).

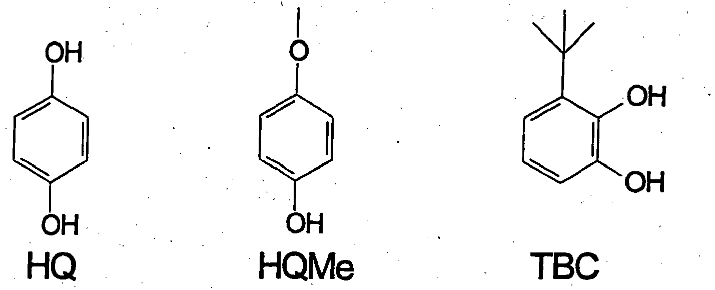

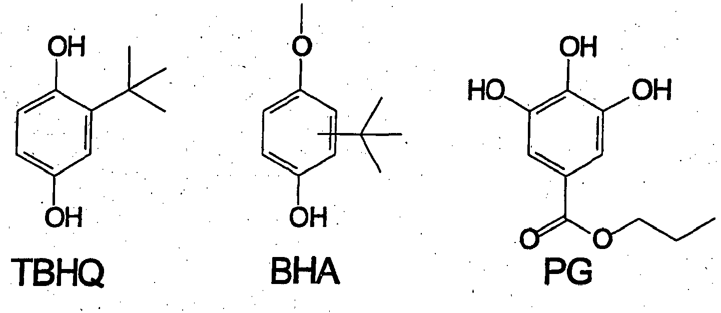

Test formulations containing different inhibitors or inhibitor concentrations were stored in Prime&Bond NT bottles (Dentsply DeTrey) at 60 °C until thermal polymerization. The bottles were daily examined by shaking the bottle, whereby the acoustical test turned out to be rather sensitive, and by taking a sample with a pipette. When polymerization seemed to have occurred or after a certain minimum storage time (20 days) the bottles were sliced open and the solution were examined visually.

According to the results of the above described Arrhenius investigation at least a thermal stability of about 11 days at 60 °C is necessary so that the dental adhesive composition may be stored at room temperature.

### Results

The Test Formulation containing different inhibitors in different amounts, was investigated regarding its thermal stability by storing these formulations at 60 °C. The samples were daily examined. In case of polymerization a gel or a solid, polymerized body was observed.

The dark shaded columns represent formulations with inhibitors, respectively inhibitor concentrations, which were polymerized after the depicted time at 60 °C. The light shaded columns represent formulations, which were not polymerized until the depicted time. Usually after 20 days the investigation was terminated.

In the comparison, hydroquinone (HQ) was used in an amount of 0.15 mol% showing some stabilization effect. However, hydroquinone is an allergenic compound and therefore undesirable for use in a generic dental composition. Hydroquinone monomethylether (HQME) as well as BHT failed to provide a sufficient thermal stability.

### (i) Hydrochinone (HQ) - Reference Inhibitor

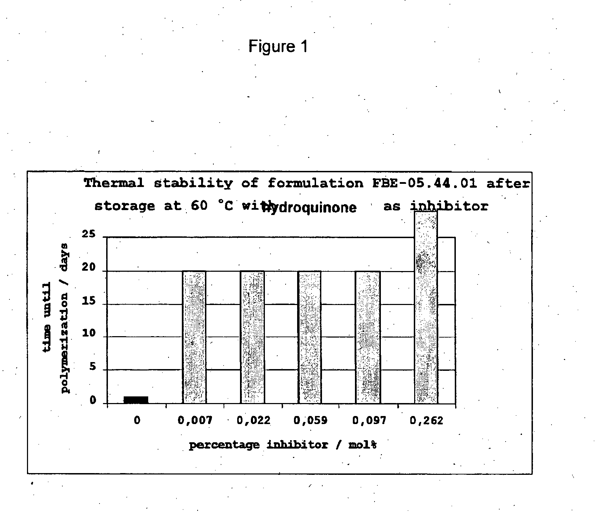

The results are shown in figure 1. (light shaded columns - formulation is not polymerized up to the recorded time; dark shaded column: formulation is polymerized after the recorded time)

### (ii) Hydrochinone Monomethylether (HQME)- Reference Inhibitor

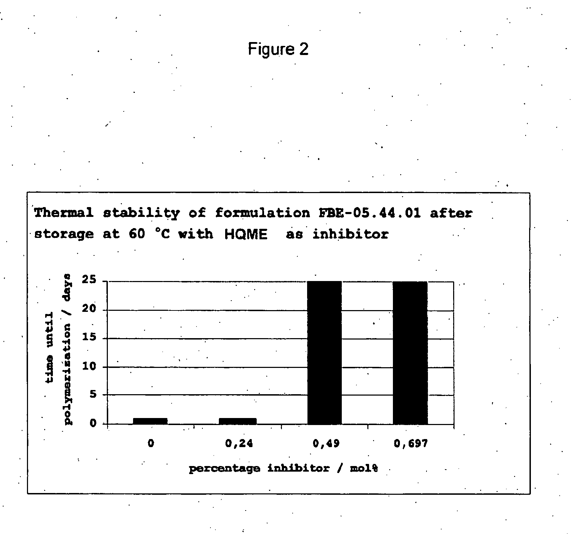

The results are shown in figure 2.

After slicing open the samples containing 0.49 and 0.697 mol% HQME, small pieces of gel were found at the bottom, which were not detected before by shaking or by the examination with the pipette.

After slicing open the sample with 0.193 mol% TBC some pieces of gel were found at the bottom, which were not detected before by shaking or by the examination with the pipette.

### (iii) Bisphenol A - Reference Inhibitor

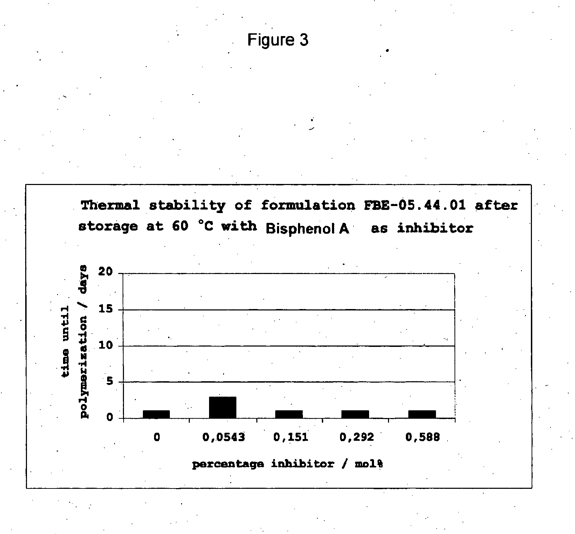

The results are shown in figure 3.

The dark shaded columns indicate that the formulation is polymerized after the recorded time.

### (iv) Propyl gallate (PG) - Reference Inhibitor

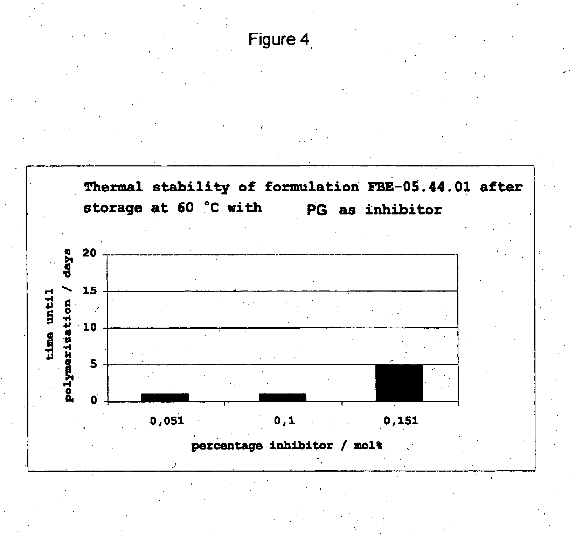

The results are shown in figure 4.

The dark shaded columns indicate that the formulation is polymerized after the recorded time

### (vii) tert.-Butylhydrochinone (TBHQ)- Inhibitor of the Invention

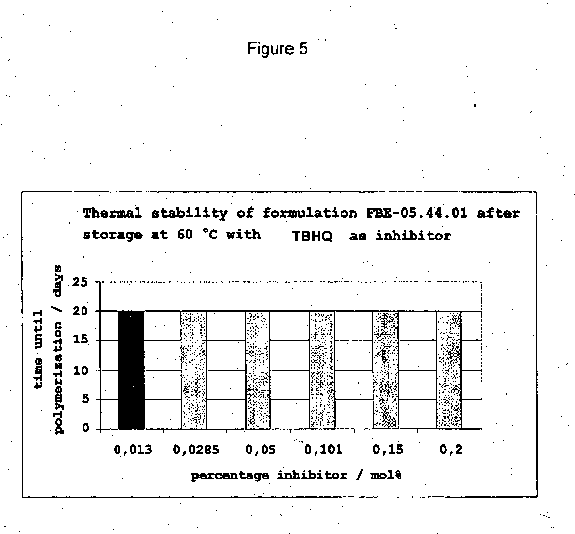

The results are shown in fig. 5.

After 14 days and after 20 days at 60 °C the bottles were sliced open, the contents was investigated and filled in a new bottle, which was stored again at 60 °C. No hints of a polymerization were found.

After 20 days at 60 °C the bottles were sliced open again and the contents was investigated. Only in case of the lowest TBHQ percentage of 0.013 mol% polymerization was found. This was not detected before by the daily examination.

### (viii) tert.-Butylhydroxyanisole (BHA) - Inhibitor of the Invention

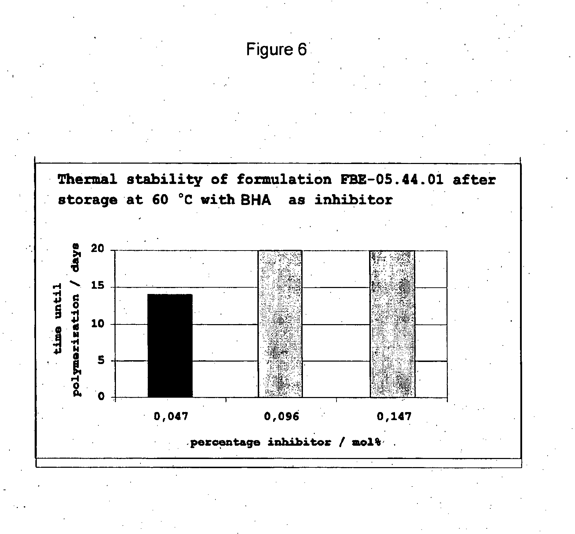

The results are shown in figure 6.

After 14 days at 60 °C all bottles were sliced open, the contents was investigated and filled in a new bottle, which was stored again at 60 °C. The sample with 0.047 mol % showed after 14 days at 60 °C some pieces of gel, which were not detected before by shaking or by the examination with the pipette. After 20 days at 60 °C the bottles were again sliced open. No indication of polymerization for the samples containing 0.096 mol% and 0.147 mol% were found. The formulation with 0.047 mol% again contains some small pieces of gel.

### Example 1

The following non-aqueous compositions were prepared with different amounts of TBHQ as an inhibitor. The compositions were stored at 65 °C. The time required for polymerization and therefore deterioration of the composition was determined.

| | **Composition / wt.-%** | | | |
|---|---|---|---|---|
| Polymerizable Resins | 67.33 | 67.29 | 67.25 | 67.21 |
| CQ/Amine | 2.67 | 2.67 | 2.67 | 2.67 |
| TBHQ | 0.04 | 0.08 | 0.12 | 0.16 |
| Nanofiller | 5.47 | 5.47 | 5.47 | 5.47 |
| t-Butanol | 24.5 | 24.5 | 24.5 | 24.5 |
| Sum | 100 | 100 | 100 | 100 |

| | **Days life to polymerization** | | | |
|---|---|---|---|---|
| **Temp.** / **°C** | **t/d** | **t/d** | **t/d** | **t/d** |
| 65 | 4 | 10 | 77 | >77 |

It was found that 0.028 - 0.2 mol% TBHQ provides a sufficient thermal stability over 20 days at 65 °C.

### Comparison Example 1

The following non-aqueous compositions were prepared with different amounts of BHT as an inhibitor. The compositions were stored at 65 °C. The time required for polymerization and therefore deterioration of the composition was determined.

| | **Composition / wt.-%** | | | |
|---|---|---|---|---|
| Polymerizable Resins | 35.35 | 66.87 | 68.11 | 53.15 |
| CQ/Amine | 1.41 | 2.67 | 1.43 | 2.12 |
| BHT | 0.26 | 0.49 | 0.49 | 0.39 |
| Nanofiller | 2.89 | 5.47 | 5.47 | 4.35 |
| t-Butanol | 60.09 | 24.50 | 24.50 | 40 |
| Sum | 100 | 100 | 100 | 100 |

| | **Days life to polymerization** | | | |
|---|---|---|---|---|
| **Temp.** / **°C** | **t/d** | **t/d** | t/**d** | **t/d** |
| 65 | 1 | 1 | 6 | 4 |

It was found that 0.028 - 0.2 mol% BHT does not provide a sufficient thermal stability over 20 days at 65 °C.

### Comparison Example 2

The following non-aqueous compositions were prepared with different amounts of PG as an inhibitor. The compositions were stored at 65 °C. The time required for polymerization and therefore deterioration of the composition was determined.

| | **Composition** / **wt.-%** | | | |
|---|---|---|---|---|
| Polymerizable Resins | 67.31 | 67.26 | 67.21 | 67.16 |
| CQ/Amine | 2.67 | 2.67 | 2.67 | 2.67 |
| PG | 0.05 | 0.10 | 0.15 | 0.2 |
| Nanofiller | 5.47 | 5.47 | 5.47 | 5.47 |
| t-Butanol | 24.5 | 24.5 | 24.5 | 24.5 |
| Sum | 100 | 100 | 100 | 100 |

| | **Days life to polymerization** | | | |
|---|---|---|---|---|
| **Temp. / °C** | **t/d** | **t/d** | **t/d** | **t/d** |
| 65 | 0-3 | 0-3 | 0-3 | 0-3 |

It was found that 0.028 - 0.2 mol% PG does not provide a sufficient thermal stability over 20 days at 65 °C.

### Example 2

0.6945 g N,N'-Bisacrylamido-N,N'-diethyl-1,3-propane, 0.2315 g 3,(4),8,(9)-bis(acrylamido methyl) tricyclo-5.2.1.0^{2,6} decane, 0.0595 g Ethyl 2-[12-dihydrogen phosphoryl-12,2-dioxatridecyl]acrylate, 0.0481 g 2-Acrylamido-2-methyl-propane-sulfonic acid, 0.0141 g camphor quinone, 0.0355 g bis (2,4,6-trimethylbenzoyl)-phenyl phosphine oxide, 0.0164 g dimethylamino benzoic acid ethyl ester and 0.003 g 2-tert-Butylhydroquinone were dissolved in a solvent mixture composed of 0.1800 g acrylic acid and 0.7200 g water.

The adhesive does not polymerise after storage for 20 days at 60 °C.

### Example 3

0.6940 g N,N'-Bisacrylamido-N,N'-diethyl-1,3-propane, 0.2313 g 3,(4),8,(9)-bis(acrylamido methyl) tricyclo-5.2.1.0^{2,6} decane, 0.0595 g Ethyl 2-[12-dihydrogen phosphoryl-12,2-dioxatridecyl]acrylate, 0.0481 g 2-Acrylamido-2-methyl-propane-sulfonic acid, 0.0141 g camphor quinone, 0.0355 g bis (2,4,6-trimethylbenzoyl)-phenyl phosphine oxide, 0.0164 g dimethylamino benzoic acid ethyl ester and 0.0011 g 2-tert.-butyl-4-methoxyphenol were dissolved in a solvent mixture composed of 0.1800 g acrylic acid and 0.7200 g water.
The adhesive does not polymerise after storage for 20 days at 60 °C.

### Comparative Example 3

0.6931 g N,N'-Bisacrylamido-N,N'-diethyl-1,3-propane, 0.2310 g 3,(4),8,(9)-bis(acrylamido methyl) tricyclo-5.2.1.0^{2,6} decane, 0.0594 g ethyl 2-[12-dihydrogen phosphoryl-12,2-dioxatridecyl]acrylate, 0.0480 g 2-Acrylamido-2-methyl-propane-sulfonic acid, 0.0141 g camphor quinone, 0.0354 g bis (2,4,6-trimethylbenzoyl)-phenyl phosphine oxide, 0.0164 g dimethylamino benzoic acid ethyl ester and 0.0026 g hydroquinone monomethyl ether were dissolved in a solvent mixture composed of 0.1800 g acrylic acid and 0.7200 g water.
The adhesive polymerises after storage for 1 day at 60 °C.

### Comparative Example 4

0.6882 g N,N'-Bisacrylamido-N,N'-diethyl-1,3-propane, 0.2294 g 3,(4),8,(9)-bis(acrylamido methyl) tricyclo-5.2.1.0^{2,6} decane, 0.0590 g Ethyl 2-[12-dihydrogen phosphoryl-12,2-dioxatridecyl]acrylate, 0.0477 g 2-Acrylamido-2-methyl-propane-sulfonic acid, 0.0140 g camphor quinone, 0.0352 g bis (2,4,6-trimethylbenzoyl)-phenyl phosphine oxide, 0.0162 g dimethylamino benzoic acid ethyl ester and 0.0103 g 2,6-di-tert.-butyl-4-cresol were dissolved in a solvent mixture composed of 0.1800 g acrylic acid and 0.7200 g water. The adhesive polymerises after storage for 2 days at 60 °C.

## Claims

1. Non-aqueous dental adhesive comprising a mixture containing
(i) one or more polymerizable monomers containing an acidic group, which are selected from 2-acrylamido-2-methylpropane sulphonic acid and phosphate esters or phosphonate derivatives of radical polymerizable alcohols or polyol derivatives,
(ii) a polymerization initiator, and
(iii) a thermal polymerization inhibitor of the following formula (I): 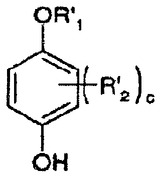 wherein
R'₁ represents
a hydrogen atom, or a saturated hydrocarbon group having 1 to 18 carbon atoms,
R'₂ is a tert.-butyl group, and
c represents an integer of from 1 to 4,
(iv) optionally an organic solvent.

2. The dental adhesive according to claim 1, which is stable at storage for at least 10 days at 60 °C.

3. The dental adhesive according to claim 1 or 2, wherein the saturated hydrocarbon group is a straight chain or branched C₁₋₁₈ alkyl group or a C₃₋₁₈ cycloalkyl group optionally substituted by one or more C₁₋₅ alkyl groups or a C₄₋₁₈ cycloalkylalkyl group optionally substituted by one or more C₁₋₅ alkyl groups.

4. The dental adhesive according to any one of the preceding claims, wherein the inhibitor is TBHQ or BHA.

5. The dental adhesive according to any one of the preceding claims, wherein the inhibitor is contained in an amount of from 0.01 to 0.5 mol%.

6. The dental adhesive according to any one of the preceding claims, wherein the aqueous mixture further contains an organic water soluble solvent selected from the group of alcohols and ketones such as ethanol, propanol, butanol, acetone, methyl ethyl ketone.

7. The dental adhesive according to any one of the preceding claims, wherein said polymerization initiator is a photo initiator such as camphor quinone.

8. The dental adhesive according to any one of the preceding claims, which further contains an inorganic filler and/or an organic filler; preferably the filler is a nanofiller.

9. The dental adhesive according to claim 1, wherein the a polymerization initiator is camphor quinone, the inhibitor is TBHQ, and the solvent is t.-butanol; and which further contains a nanofiller.

10. Use of a compound of the following formula (I): 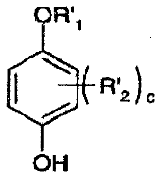 wherein R'₁
represents
a hydrogen atom, or a saturated hydrocarbon group having 1 to 18 carbon atoms.
R'₂ is a tert.-butyl group, and
c represents an integer of from 1 to 4,
as a thermal polymerization inhibitor in a dental composition according to one of claims 1 to 9.

## Patentansprüche

1. Nichtwässriges Dentaladhäsiv, umfassend ein Gemisch, enthaltend
(i) ein oder mehrere polymerisierbare Monomere, enthaltend eine saure Gruppe, die ausgewählt sind aus 2-Acrylamido-2-methylpropansulfonsäure und Phosphatestern oder Phosphonatderivaten radikalisch polymerisierbarer Alkohole oder Polyolderivate,
(ii) ein Polymerisationsinitiator, und
(iii) ein thermischer Polymerisationsinhibitor der folgenden Formel (I): 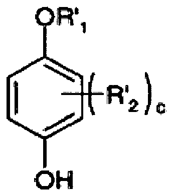 wobei
R'₁ für ein Wasserstoffatom oder eine gesättigte Kohlenwasserstoffgruppe mit 1 bis 18 Kohlenstoffatomen steht,
R'₂ für eine *tert*.-Butylgruppe steht, und
c für eine ganze Zahl von 1 bis 4 steht,
(iv) gegebenenfalls ein organisches Lösungsmittel.

2. Das Dentaladhäsiv nach Anspruch 1, das bei Lagerung bei 60°C für mindestens 10 Tage stabil ist.

3. Das Dentaladhälsiv nach Anspruch 1 oder 2, wobei die gesättigte Kohlenwasserstoffgruppe für eine geradkettige oder verzweigte C₁₋₁₈ Alkylgruppe, für eine C₃₋₁₈ Cycloalkylgruppe, die gegebenenfalls mit einer oder mehreren C₁₋₅ Alkylgruppen substituiert ist, oder für eine C₄₋₁₈ Cycloalkylalkylgruppe, die gegebenenfalls mit einer oder mehreren C₁₋₅ Alkylgruppen substituiert ist, steht.

4. Das Dentaladhäsiv nach einem der vorstehenden Ansprüche, wobei der Inhibitor TBHQ oder BHA ist.

5. Das Dentaladhäsiv nach einem der vorstehenden Ansprüche, wobei der Inhibitor in einer Menge von 0.01 bis 0.5 Mol-% enthalten ist.

6. Das Dentaladhäsiv nach einem der vorstehenden Ansprüche, wobei das Gemisch ferner enthält ein organisches wasserlösliches Lösungsmittel, ausgewählt aus der Gruppe der Alkohole und Ketone, wie Ethanol, Propanol, Butanol, Aceton, Methylethylketon.

7. Das Dentaladhäsiv nach einem der vorstehenden Ansprüche, wobei der Polymerisationsinitiator ein Photoinitiator, wie Kampferchinon, ist.

8. Das Dentaladhäsiv nach einem der vorstehenden Ansprüche, das ferner enthält einen anorganischen Füllstoff und/oder einen organischen Füllstoff, vorzugsweise ist der Füllstoff ein Nanofüllstoff.

9. Das Dentaladhäsiv nach Anspruch 1, wobei der Polymerisationsinitiator Kampferchinon ist, der Inhibitor TBHQ ist und das Lösungsmittel t.-Butanol ist, und das ferner einen Nanofüllstoff enthält.

10. Verwendung einer Verbindung der folgenden Formel (I): 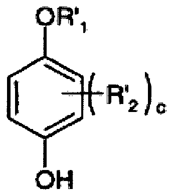 wobei
R'₁ für ein Wasserstoffatom oder eine gesättigte Kohlenwasserstoffgruppe mit 1 bis 18 Kohlenstoffatomen steht,
R'₂ für eine *tert*.-Butylgruppe steht, und
c für eine ganze Zahl von 1 bis 4 steht,
als thermischer Polymerisationsinhibitor in einer Dentalzusammensetzung nach einem der Ansprüche 1 bis 9.

## Revendications

1. Adhésif dentaire non aqueux comprenant un mélange contenant :
(i) un ou plusieurs monomères polymérisables contenant un groupe acide, qui sont choisis entre l'acide 2-acrylamio-2-méthylpropane-sulfonique et les esters phosphates ou dérivés de phosphonates d'alcools ou de dérivés de polyols aptes à la polymérisation radicalaire,
(ii) un initiateur de polymérisation, et
(iii) un inhibiteur de polymérisation thermique répondant à la formule (I) suivante : 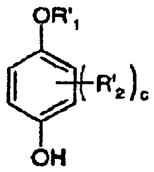 dans laquelle
R^{'}₁ représente un atome d'hydrogène, ou un groupe hydrocarboné saturé ayant 1 à 18 atomes de carbone,
R^{'}₂ représente un groupe tertiobutyle, et
c représente un nombre entier de 1 à 4,
(iv) facultativement, un solvant organique.

2. Adhésif dentaire suivant la revendication 1, qui est stable au stockage pendant au moins 10 jours à 60°C.

3. Adhésif dentaire suivant la revendication 1 ou 2, dans lequel le groupe hydrocarboné saturé est un groupe alkyle en C₁ à C₁₈ à chaîne droite ou ramifié ou un groupe cycloalkyle en C₃ à C₁₈ facultativement substitué avec un ou plusieurs groupes alkyle en C₁ à C₅ ou un groupe cycloalkylalkyle en C₄ à C₁₈ facultativement substitué avec un ou plusieurs groupes alkyle en C₁ à C₅.

4. Adhésif dentaire suivant l'une quelconque des revendications précédentes, dans lequel l'inhibiteur est la TBHQ ou la BHA.

5. Adhésif dentaire suivant l'une quelconque des revendications précédentes, dans lequel l'inhibiteur est présent en une quantité de 0,01 à 0,5 % en moles.

6. Adhésif dentaire suivant l'une quelconque des revendications précédentes, dans lequel le mélange aqueux contient en outre un solvant organique soluble dans l'eau choisi dans le groupe des alcools et des cétones, tels que l'éthanol, le propanol, le butanol, l'acétone et la méthyl-éthylcétone.

7. Adhésif dentaire suivant l'une quelconque des revendications précédentes, dans lequel ledit initiateur de polymérisation est un photoinitiateur tel que la camphoquinone.

8. Adhésif dentaire suivant l'une quelconque des revendications précédentes, qui contient en outre une charge inorganique et/ou une charge organique ; la charge étant de préférence une nanocharge.

9. Adhésif dentaire suivant la revendication 1, dans lequel l'initiateur de polymérisation est la camphoquinone, l'inhibiteur est la TBHQ et le solvant est le tertiobutanol, et qui contient en outre une nanocharge.

10. Utilisation d'un composé répondant à la formule (I) suivante : 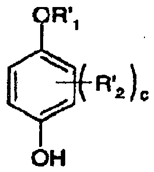 dans laquelle
R^{'}₁ représente un atome d'hydrogène, ou un groupe hydrocarboné saturé ayant 1 à 18 atomes de carbone,
R^{'}₂ représente un groupe tertiobutyle, et
c représente une nombre entier de 1 à 4,
comme inhibiteur de polymérisation thermique dans une composition dentaire suivant l'une des revendications 1 à 9.
